(19) Europäisches Patentamt
European Patent Office
Office européen des brevets

(11) Publication number: **0 493 378 A1**

(12) **EUROPEAN PATENT APPLICATION**

(21) Application number: **92200531.9**

(51) Int. Cl.5: **A61K 31/70**, A61K 31/52

(22) Date of filing: **21.08.86**

This application was filed on 24 - 02 - 1992 as a divisional application to the application mentioned under INID code 60.

(30) Priority: **26.08.85 US 769016**

(43) Date of publication of application: **01.07.92 Bulletin 92/27**

(60) Publication number of the earlier application in accordance with Art.76 EPC: **0 216 510**

(84) Designated Contracting States: **AT BE CH DE FR GB IT LI LU NL SE**

(71) Applicant: **THE UNITED STATES OF AMERICA, as represented by THE SECRETARY, U.S. DEPARTMENT OF COMMERCE**
**5285 Port Royal Road**
**Springfield Virginia 22161(US)**

(72) Inventor: **Mitsuya, Hiroaki**
**259 Congressional Lane T-2**
**Rockville, Maryland 20852(US)**
Inventor: **Border, Samuel**
**6004 Rossmore Drive**
**Bethesda, Maryland 20814(US)**

(74) Representative: **Stebbing, Peter John Hunter et al**
**F.J. CLEVELAND & COMPANY 40-43, Chancery Lane**
**London WC2A 1JO(GB)**

(54) **Use of 2',3'-dideoxyguanosine for the treatment of AIDS.**

(57) The present invention relates to a method of alleviating the cytopathic effects of HTLV-III virus on cells which comprises contacting the virus with an effective amount of a purine base, which is 2',3'-dideoxyguanosine, or a phosphate thereof.

FIG.1.

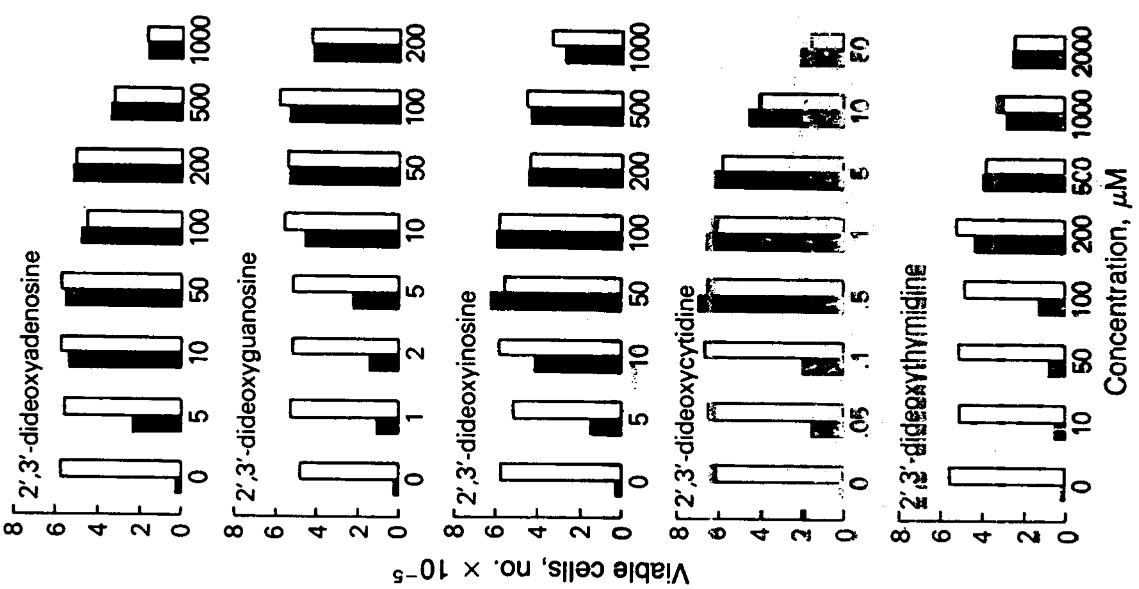


EP 0 493 378 A1

The present invention relates to the inhibition of in vitro infectivity and cytopathic effect of HTLV-III/LAV by 2',3'-dideoxyguanosine.

Since 1983 it has developed that the virus HTLV-III is the etiologic agent of acquired immunodeficiency syndrome (AIDS) and related diseases. Medicine is currently endeavouring to find agents which will check the ravages of the disease. In this connection, a number of patent applications have been filed naming as a co-inventor Robert C. Gallo; one patent has issued, U.S. Patent No. 4,520,113, which mainly relates to the detection of antibodies in the sera of AIDS and PRE-AIDS patients.

Further in Science (1983) Vol 220 P 506-508 Montagnier similarly discloses the investigaton and isolation of an agent subsequently defined as HTLV-III.

The present invention, however, lies in a method of alleviating the effects of HTLV-III by the in vitro contacting of the virus with a particular purine base, namely, 2',3'-dideosyguanosine, and related mono- and tri-phosphates of this base.

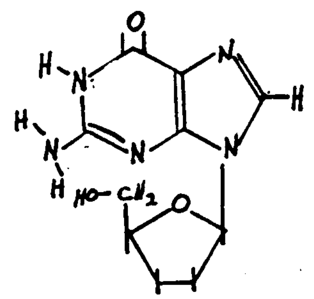

ddG

The following references are relevant to determining the state of the art prior to the present invention.

Furmanski, et al, "Inhibition of 2',3'-Dideoxy-thymidine of Retroviral Infection of Mouse and Human Cells, " Cancer Letters , 8:307-315, 1980;

Wager, et al, "Effects of 2',3'-Dideoxynucleosides on Mammalian Cells and Viruses, " Journal of Cellular Physiology, 121:402-408 (1984);

Rosen, et al, "The Location of Cis-Acting Regulatory Sequences in the Human T Cell Lymphotropic Virus Type III (HTLV-III/LAV) Long Terminal Repeat," Cell, 41:813-823, July 1985;

Time, August 12, 1985, p. 42 (column 3).

US Patent No. 4,434,788 to Nakataugawa shows the utilization of 3'-deoxyguanosine and 3'-deoxyuridine as anti-tumor agents; and

US Patent No. 4,081,534 to Elion et al shows various purine derivatives utilized against HSV.

The uniqueness of the HTLV-III virus and its resistance to treatment by known antiviral agents has been stated in Time magazine as follows: "Research conducted by Harvard's Haseltine and published in the July issue of the journal Cell [Cell, 41:813-823, 1985] reveals that the virus has a unique genetic component that allows it to reproduce itself a thousand times as fast as any other kind of virus. The mechanism for this reproduction is "one of the biggest effects I've seen in biology", says Haseltine. "It helps explain why AIDS is such a devastating disease and why it can spread so fast". In the process of rampant replication, the AIDS virus destroys its home, the T cell. Thus it is a peculiar feature of this disease that as it progresses, the helper T cells disappear and so does the virus. By then, however, the patient is invariably beyond recovery".

EP-A-0206497 is an intervening publication which reveals in the relevant priority document the utilization of a 2',3'-dideoxynucleoside exemplified solely by dideoxyribofuranoside. The use of the compound of our invention is not supported in this disclosure.

The Journal of Cellular Physiology 1984, Vol 121, No. 2, November 1984 reveals that 2',3'-dideoxyguanosine inhibits the growth of MuLV virus at about 50 $\mu$m. The disclosure is silent on the application of dideoxyguanosine to alleviate the symptoms of HTLV-III.

It has been noted that the chronicity of infection and the propensity of the virus to infect the brain make it necessary to develop new classes of remedies and drugs which have the potential for oral administration and penetration across the blood-brain barrier. In this study, the capacity of purine and pyrimidine nucleoside derivatives to inhibit the infectivity and cytopathic effect of HTLV-III/LAV in vitro were explored. With the ribose moiety of the molecule in a 2',3'-dideoxy-configuration, the guanosine nucleosides tested suppressed the virus. In general, it was observed that complete suppression of the virus occurs at doses

that were 10- to 20-fold lower than those needed to inhibit the proliferation of the target T-cells and the immune reactivity of normal T-cells in vitro. An analysis of five adenosine congeners, which differed only in the sugar moiety, revealed that reduction (an absence of hydroxyl determinants) at both the 2' and 3' carbons of the ribose was necessary for an anti-viral effect, and an additional reduction at the 5' carbon nullified the anti-viral activity.

## Brief Description of the Drawings

Figure 1 illustrates the potent protective effect of 2',3'-dideoxynucleosides on the survival and growth of ATH8 cells when exposed to HTLV-III/LAV.

Figure 2 illustrates the expression of the HTLV-III/LAV p24 gag protein in H9 cells.

## Detailed Description of the Drawings

In Figure 1, which shows the inhibition of cytopathic effect of HTLV-III/LAV by 2',3'-dideoxynucleosides against ATH8 cells, $2x10^5$ ATH8 cells were preexposed to polybrene, exposed to HTLV-$III_B$ (2,000 virus 2,000 virus particles/cell) in culture tubes (solid columns) in the presence or absence of various concentrations of 2',3'-dideoxyadenosine, 2',3'-dideoxyinosine, 2',3'-dideoxyguanosine (P.L. Biochemicals Inc.) 2',3'-dideoxycytidine, or 2',3'-dideoxythymidine. Control cells (open columns) were similarly treated but were not exposed to the virus. On day 5, total viable cells were counted as described in Example 1.

In Figure 2, which shows the inhibition of the infectivity and replication of HTLV-III/LAV in H9 cells by 2',3'-dideoxynucleosides, $10^5$ H9 cells were exposed to various concentrations of 2',3'-dideoxynucleosides for 4 hours, then to 2 $\mu$g/ml polybrene for 30 minutes, pelleted, and exposed to HTLV-$III_B$ (3,000 virus particles/cell) for 1.5 hours. Cells were resuspended in fresh complete medium and cultured in tubes at 37°C in 5% $CO_2$-containing humidified air. The cells were continuously exposed to 2',3'-dideoxynucleosides. On days 8 (left), 9 (middle), and 10 (right) in culture, the percentage of the target H9 cells expressing p24 gag protein of HTLV-III/LAV was determined by indirect immunofluorescence microscopy by using anti-HTLV-III/LAV p24 murine monoclonal antibody (M26 provided by Drs. F.D. Veronese and R.C. Gallo).

## Specific Description

In the system, HTLV-III/LAV (as cell-free virus) exerts a profound cytopathic effect on the target T-cells by day 4 in culture, and by day 10, 98% of the cells are killed by the virus. The killing of cells can be monitored quantitatively as a function of the starting dose of virus (Table 1). When ATH8 cells are used in a 7-day assay, 5 virus particles/cell represented the minimum cytopathic dose of virus. In the experiments reported below, 2,000 or 3,000 virus particles/cell were used in order to test various compounds under conditions of substantial virus excess.

Figure 1 illustrates the potent protective effect of 2',3'-dideoxynucleosides on the survival and growth of ATH8 cells when exposed to HTLV-III/LAV. It has been found that concentrations greater than 0.5 $\mu$M of 2',3'-dideoxycytidine completely protected ATH8 cells and enabled them to survive and grow. This compound exhibited a strong anti-viral effect at doses that were 10- to 20-fold lower than those that inhibited growth of the target cells when no virus was present. In the present experimental conditions, 2',3'-dideoxythymidine required relatively high concentrations to exert a protective effect, and unlike the other comparable dideoxynucleosides tested, its capacity to nullify the cytopathic effect of the virus was lost on day 10 of the culture (Figure 1 (bottom)).

These anti-viral effects were confirmed in a different system, using the expression of the HTLV-III/LAV p24 gag protein in H9 cells (Figure 2). The H9 cells are relatively resistant to the cytopathic effect of HTLV-III/LAV, and p24 gag protein expression following exposure to HTLV-III/LAV virions may be used as an index of viral infectivity and replication in vitro. The 2',3'-dideoxy-derivatives of cytidine were potent inhibitors of viral replication in the system, while 2',3'-dideoxythymidine required relatively higher concentrations to mediate an anti-viral effect, and this compound allowed a resumption of viral replication by day 10 of culture; (Figure 2 (bottom)).

Also tested were the effects of the various 2',3'-dideoxynucleosides on the antigen-specific and lectin-induced reactivity of normal lymphocytes in vitro (Table 2). A normal clone (TM3) of tetanus-toxoid specific helper/inducer T-cells was used to monitor the effects of the compounds on antigen-driven activation, and normal circulating lymphocytes were used to monitor effects on pokeweed mitogen and phytohaemagglutinin-driven activation. Concentrations of these compounds, including those that were 10- to

20-fold higher than those necessary to block the in vitro infectivity and cytopathic effect of HTLV-III/LAV left the in vitro immune reactivity of normal lymphocytes basically intact.

The mechanisms by which 2',3'-dideoxynucleosides suppress the replication of HTLV-III/LAV are not known. It is known that the 5'-triphosphate product of 2',3'-dideoxyguanosine, can inhibit cellular DNA polymerases beta and gamma, as well as viral reverse transcriptase, but not mammalian DNA polymerase alpha. DNA polymerase alpha is assumed to be the key DNA synthetic enzyme for DNA replication during cell division, and it also has a role in DNA repair. Of interest, Herpes simplex type I DNA polymerase is reported to be as resistant to 2',3'-dideoxythymidine as cellular DNA polymerase alpha. Unphosphorylated dideoxynucleosides have rather negligible effects on the growth of cultured mammalian cells (a phenomenon which is confirmed here in human T-cells). This is believed to be so because of inefficient intracellular conversion to the corresponding 5'-triphosphates coupled with the resistance of DNA polymerase alpha to low levels of the 5'-triphosphates.

Example 1

With reference to Table 1, a tetanus toxoid-specific T-cell line, established by repeated cycles of stimulation with antigen as described in Mitsuya, et al, Science, 225:1484-1486 (1984), was cloned in the presence of lethally irradiated (12,000 rad) human T-lymphotropic virus type I (HTLV-I)-producing MJ-tumor cells in 96-well microtiter culture plates (Costar, Cambridge, MA). Clone ATH8 (obtained from a culture plated at 0.5 cells per well) was selected for drug screening on the basis of its rapid growth (in the presence of interleukin-2) and exquisite sensitivity to the cytopathic effect of HTLV-III/LAV. ATH8 clone bears several distinct copies of HTLV-I in its genome when assessed by Southern blot hybridization using a radiolabelled HTLV-I cDNA probe but does not produce detectable amounts of HTLV-I p24 gag protein. $10^5$ ATH8 cells were pre-exposed to 2 μg/ml polybrene for 30 minutes, pelleted, exposed to various amounts of HTLV-$III_B$, resuspended in 2 ml complete medium (RPMI supplemented with 15% undialysed, heat inactivated fetal calf serum, 4mM Lu-glutamine, $5x10^{-5}$ 2-mercaptoethanol, 50 U/ml penicillin, and 50 μg/ml streptomycin) containing 15% (vol/vol)-interleukin2 (lectin-depleted; Cellular Products Inc., Buffalo, NY), and incubated in culture tubes (3033, Falcon, Oxnard, CA) at 37°C in 5% $CO_2$-containing humidified air. On day 7, the total viable cells were counted by the trypan blue dye exclusion method. Table 1 shows the cytopathic effect of HTLV-III/LAV on the ATH8 cells; data are expressed as the arithmetic means ±1 standard deviation for duplicate determinations.

Table 1

| Cytopathic Effect of HTLV-III/LAV on ATH8 Cells | |
|---|---|
| Number of HTLV-$III_B$ Virus Particles/Cell | Number of Viable ATH8 Cells ($x10^5$) |
| 0 | 3.37 ± 0.1 |
| 0.05 | 3.36 ± 0.04 |
| 0.5 | 3.26 ± 0.15 |
| 5 | 1.97 ± 0.2 |
| 50 | 1.78 ± 0.16 |
| 500 | 0.37 ± 0.02 |
| 5,000 | 0.30 ± 0.01 |

Table 2

Effect of 2',3'-Dideoxynucleosides on the in vitro Immune Reactivity of Normal Lymphocytes

| Responder Cells | None | Guanosine[1] | | None[4] | Cytidine[4] | | Tymidine[4] | |
|---|---|---|---|---|---|---|---|---|
| | | 10 | 100 | | 1 | 10 | 200 | 2,000 |
| Clone TM3[2] | 75+6 | 66+5 | 77+1 | 12+1 | 13+1 | 8+1 | 9+1 | 9+1 |
| PBM + PHA[3] | 310+39 | 350+4 | 469+9 | 82+1 | 101+1 | 86+3 | 86+6 | 77+8 |
| PBM + PWM[3] | 63+2 | 73+8 | 62+5 | 24+1 | 23+3 | 15+3 | 31+1 | 31+1 |

1 - All nucleosides tested are of 2',3'-dideoxy-configuration. Concentrations (µM) of each 2',3'-dideoxynucleoside bring about virtually complete inhibition of the cytopathic effect of HTLV-III/LAV and the viral p24 expression (except 2',3'-dideoxythymidine); see Figures. $^3$H-thymidine incorporation was used as an indicator of activation of responder cells, unless otherwise indicated.

2. - $5x10^4$ normal helper/inducer TM3 cells were stimulated with tetanus toxoid (0.6 limiting flocculation units/ml) plus $10^5$ irradiated (4,000 rad) autologous peripheral blood mononuclear cells (PBM) and cultured for 72 hours in the presence or absence of the 2',3'-dideoxynucleoside. After exposure to 0.5 µCi $^3$H-thymidine or 1 µCi $^3$H-uridine for the final 5 hours, cells were harvested onto glass fibers and the incorporated radioactivity was counted. Data are expressed as the arithmetic mean counts per minute ($x10^3$) ±1 standard deviation triplicate determinations.

3. - $10^6$ PBM from a healthy individual were stimulated with phytohaemagglutinin (PHA) or pokeweed mitogen (PWM) and cultured for 72 hours and treated as described above.

4. - $^3$H-uridine incorporation was used as an indicator of activation of the responder cells.

## Claims

1. The use of an effective amount of a 2',3'-dideoxyguanosine or a mono- or tri- phosphate thereof in the preparation of a medicament for the treatment of AIDS and AIDS related diseases.

2. The use according to claim 1 wherein the medicament is in dosage form adapted to give an in vivo concentration of 10 to 100 µm.

FIG.1.

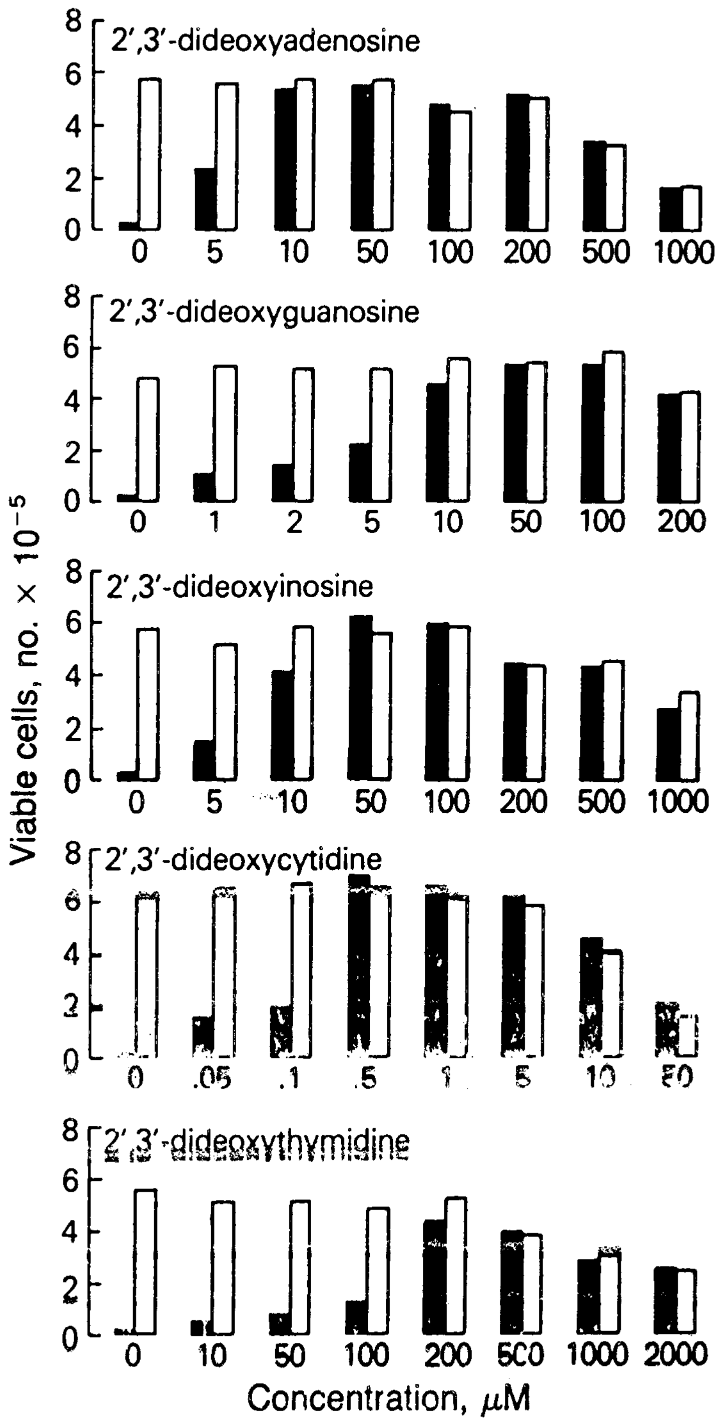
Viable cells, no. × 10⁻⁵
2',3'-dideoxyadenosine
0 5 10 50 100 200 500 1000
2',3'-dideoxyguanosine
0 1 2 5 10 50 100 200
2',3'-dideoxyinosine
0 5 10 50 100 200 500 1000
2',3'-dideoxycytidine
0 .05 .1 .5 1 5 10 50
2',3'-dideoxythymidine
0 10 50 100 200 500 1000 2000
Concentration, μM

FIG.2

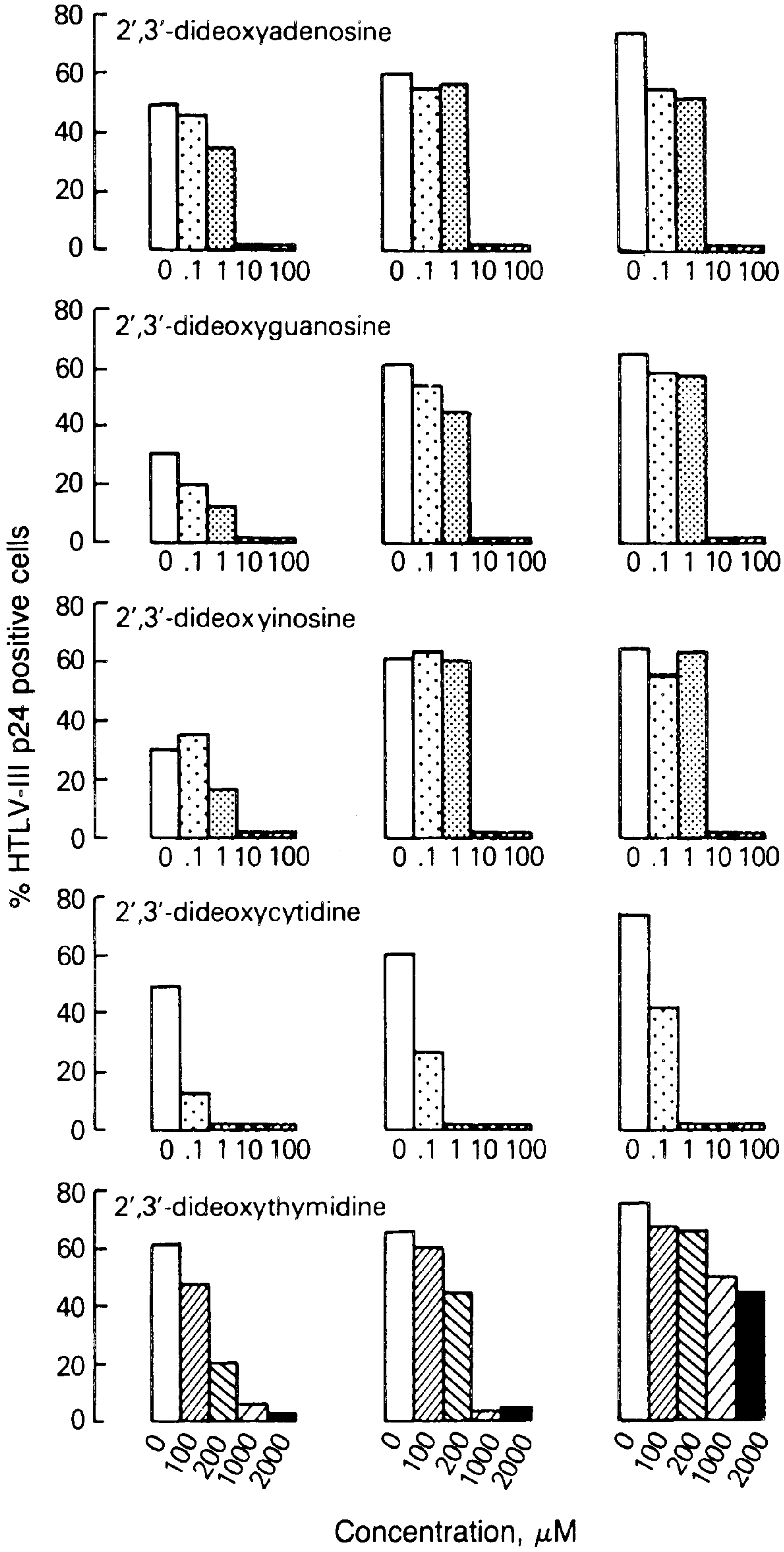
% HTLV-III p24 positive cells
2',3'-dideoxyadenosine
2',3'-dideoxyguanosine
2',3'-dideoxyinosine
2',3'-dideoxycytidine
2',3'-dideoxythymidine
0 .1 1 10 100
0 100 200 1000 2000
Concentration, µM

European Patent Office

# EUROPEAN SEARCH REPORT

Application Number

EP 92 20 0531

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,E | EP-A-0 206 497 (THE WELLCOME FOUNDATION LTD)<br>* Whole document *<br>--- | 1,2 | A 61 K 31/70<br>A 61 K 31/52 |
| P,X | PROC. NATL. ACAD. SCI. USA, vol. 83, March 1986, pages 1911-1915; H. MITSUYA et al: Inhibition of the in vitro infectivity and cytopathic effect of human T-lymphotrophic virus type III/lymphadenopathy-associated virus (HTLV-III/LAV) by 2',3'-dideoxynucleosides"<br>* Whole article *<br>--- | 1,2 | |
| D,Y | JOURNAL OF CELLULAR PHYSIOLOGY, vol. 121, no. 2, 1984, pages 402-408, Alan R. Liss, Inc.; M.A. WAQAR et al.: Effects of 2',3'-dideoxynucleosides on mammalian cells and viruses"<br>* Whole article *<br>--- | 1,2 | TECHNICAL FIELDS SEARCHED (Int. Cl.5) |
| Y | PHARM. RES., no. 1, 1984, pages 11-18; R.K. ROBINS: The potential of nucleotide analogs as inhibitors of Retroviruses and tumors"<br>* Whole article *<br>----- | 1,2 | A 61 K |

The present search report has been drawn up for all claims

| Place of search | Date of completion of the search | Examiner |
|---|---|---|
| THE HAGUE | 03-04-1992 | ORVIZ DIAZ P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document